(19) Europäisches Patentamt
European Patent Office
Office européen des brevets

(11) **EP 1 827 223 B1**

(12) **FASCICULE DE BREVET EUROPEEN**

(45) Date de publication et mention
de la délivrance du brevet:
**21.01.2015 Bulletin 2015/04**

(21) Numéro de dépôt: **05850594.2**

(22) Date de dépôt: **22.12.2005**

(51) Int Cl.:
*A61B 5/00* *(2006.01)* *A61B 5/053* *(2006.01)*

(86) Numéro de dépôt international:
**PCT/FR2005/003255**

(87) Numéro de publication internationale:
**WO 2006/070124 (06.07.2006 Gazette 2006/27)**

(54) **APPAREIL DE MESURE DE LA COMPOSITION CORPORELLE**

VORRICHTUNG ZUR MESSUNG DER KÖRPERZUSAMMENSETZUNG

APPARATUS FOR MEASURING BODY COMPOSITION

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.12.2004 FR 0413845**

(43) Date de publication de la demande:
**05.09.2007 Bulletin 2007/36**

(73) Titulaire: **SEB S.A.**
**69130 Ecully (FR)**

(72) Inventeur: **SIMOND, Bénédicte**
**F-74150 Bloye (FR)**

(74) Mandataire: **Kiehl, Hubert**
**SEB Développement**
**Les 4 M-Chemin du Petit Bois**
**B.P. 172**
**69134 Ecully Cedex (FR)**

(56) Documents cités:
**EP-A- 1 095 613 EP-A- 1 219 937**
**EP-A- 1 386 581 US-A1- 2004 199 057**

EP 1 827 223 B1

## Description

La présente invention concerne un appareil de mesure de la composition corporelle comportant un circuit électrique de mesure associé à une unité de calcul de l'impédance bioélectrique d'une personne.

La mesure de la composition corporelle donne des indications sur l'état de santé d'une personne. Plus particulièrement, la connaissance de la quantité de masse grasse de l'organisme et de son évolution dans le temps constitue une aide efficace lors d'un régime. Parmi les méthodes de mesure de la masse grasse, on relève la mesure de l'impédance du corps humain corrélée avec la quantité d'eau dans le corps : le corps humain est composé de masse maigre, constituée par les muscles, les os, les organes, les tissus et de masse grasse. L'eau constitue environ 70% de la masse maigre, alors que la masse grasse n'en contient presque pas. Par conséquent, en mesurant l'impédance bioélectrique du corps humain, on peut calculer, moyennant quelques paramètres d'ajustement, notamment le poids total d'une personne, la masse grasse et la masse maigre de la personne. La mesure de l'impédance bioélectrique est généralement basée sur la conductibilité des tissus lors du passage d'un courant alternatif bref de haute fréquence et faible intensité.

Les appareils fonctionnant sur ce principe calculent et affichent la composition corporelle de l'individu (masse grasse et masse maigre) exprimée en pourcentage du poids total ou en valeur absolue. Certains appareils de mesure de la composition corporelle mesurent en même temps le poids et affichent de manière graphique un suivi dans le temps des différents paramètres mesurés, par exemple, en mettant le poids et la masse grasse en ordonnée et le temps en abscisse.

Un tel appareil est décrit dans le document WO 99/52425, où l'appareil de mesure de la composition corporelle comporte un dispositif d'affichage numérique et graphique des valeurs mesurées et enregistrées par l'appareil. L'afficheur comporte une première zone d'affichage qui affiche sous forme numérique les valeurs mesurées du poids et celles de la composition corporelle, ainsi qu'une deuxième zone d'affichage graphique qui montre sous forme d'histogramme la tendance de variation dans le temps, par semaine ou par mois, de la moyenne du poids, respectivement de la composition corporelle. Toutefois, cet appareil ne permet que la mesure et le suivi de chaque paramètre de façon indépendante. Cette méthode est simple, mais pas très efficace dans l'interprétation des résultats, car elle ne tient pas compte des interactions qui existent entre les paramètres mesurés, plus particulièrement entre le poids et la masse grasse.

Une solution a été apportée par le document EP 1 201 187 qui décrit un appareil de mesure de l'impédance bioélectrique et du poids d'un individu comportant des moyens de calcul de l'indice corporel, de l'indice de masse grasse et de l'indice de masse maigre, chacun des paramètres mesurés étant alors rapporté à la taille au carré. L'appareil est également muni d'un afficheur qui fournit une représentation graphique en prenant, en ordonnée, l'indice de masse grasse ou l'indice de masse maigre et, en abscisse, l'indice corporel. L'afficheur utilisé est du type matriciel et comporte plusieurs cases définissant chacune un type prédéterminé de corpulence. A chaque mesure, un voyant met en surbrillance l'une des cases informant l'utilisateur de sa corpulence, ce dernier devant alors lire les deux messages correspondant aux projections du point en surbrillance sur les deux axes de coordonnées. Toutefois, cette information est valable à l'instant de la mesure et elle est faite par rapport à des standards prédéfinis. Ce document donne certes des informations quant à la corpulence d'un individu en tenant compte de la relation qui existe entre l'indice corporel et les indices de masse grasse et de masse maigre, mais ne prend pas en compte l'évolution dans le temps de cette interaction.

Par ailleurs, le document US 2004/0199057 apporte une solution qui semble plus compréhensible pour l'utilisateur, car l'appareil communique avec un ordinateur à l'écran duquel est affiché un message accompagné d'une animation renseignant l'utilisateur sur l'évolution de chacun des paramètres de la composition corporelle mesuré. Toutefois, une telle solution est coûteuse, car elle fait appel à une technique complexe de communication entre plusieurs appareils et un ordinateur.

Une solution simplifiée a été proposée dans le document EP 1 386 581 où tous les messages d'évolution de divers paramètres de la composition corporelle de l'individu sont affichés à l'écran même de l'appareil. L'inconvénient d'un tel affichage est qu'il n'est pas facile à comprendre, car il annonce plusieurs messages pour un seul paramètre, plusieurs paramètres étant affichés à l'écran en même temps. Par conséquent, l'utilisateur doit lui-même effectuer la corrélation entre les valeurs affichées des différents paramètres ou alors il doit lire un conseil affiché à l'écran. Cet appareil doit alors disposer d'un écran d'affichage d'assez grandes dimensions, donc coûteux, sans rendre pour autant le message de conseil lisible à distance, lorsque l'utilisateur se tient debout sur le plateau de mesure de l'appareil.

En effet, après avoir effectué une mesure d'un paramètre physiologique, on s'interroge sur son évolution dans le temps. Par ailleurs, il est généralement connu que le poids ou la composition corporelle d'un individu sont des paramètres qui peuvent varier de manière naturelle dans le temps et avec une plage de variation plus ou moins importante selon les individus. Notamment le poids peut varier parfois de plusieurs kg d'un jour à l'autre. Des fluctuations de la masse grasse peuvent également être observées d'un jour à l'autre. De telles variations du poids ou de la masse grasse sont dues à des facteurs tels que l'activité physique, le stress, le cycle hormonal, des excès alimentaires ponctuels, etc., et il devient alors nécessaire de connaître l'évolution de plusieurs paramètres dans le temps et leur interaction.

Le but de la présente invention est de remédier au moins en partie aux inconvénients susmentionnés et de proposer un appareil de mesure de la composition corporelle apte à évaluer la relation entre l'évolution dans le temps de la composition corporelle de l'individu, notamment de sa masse grasse ou masse maigre ou masse musculaire par rapport à son poids.

Un autre but de l'invention est un appareil de mesure de la composition corporelle apte à représenter le résultat de l'analyse de corrélation entre les paramètres mesurés de manière simple et facile d'interprétation même pour un utilisateur non avisé.

Un autre but de l'invention est un appareil de mesure de la composition corporelle apte à évaluer la corrélation entre les vraies tendances d'évolution de deux paramètres de la composition corporelle.

Un autre but de l'invention est un appareil de mesure de la composition corporelle qui soit d'une construction simplifiée, tout en étant d'un fonctionnement fiable, et qui puisse être fabriqué en grande série pour un moindre coût.

Ces buts sont atteints avec un appareil de mesure de la composition corporelle, tel que défini dans la revendication1, comportant des moyens de mesure d'une impédance bioélectrique, des moyens de mesure du poids, des moyens de saisie des données corporelles personnelles, des moyens de mise en mémoire et de calcul des valeurs de la composition corporelle reliés à des moyens de communication des résultats, où lesdits moyens de calcul comportent des moyens de comparaison qui comparent les dernières valeurs mesurées pour deux paramètres différents de la composition corporelle d'un individu à des valeurs antérieures de ces paramètres mesurées pour un même individu, du fait que les moyens de calcul comportent des moyens d'analyse des deux résultats issus des moyens de comparaison qui envoient un signal auxdits moyens de communication pour émettre un message unique représentatif de l'évolution conjointe des deux paramètres.

Par paramètre de la composition corporelle on comprend : le poids, la masse grasse, la masse maigre, la masse musculaire, la masse hydrique, la masse osseuse, la masse viscérale.

Un tel appareil de mesure de la composition corporelle d'un individu permet donc déjà d'estimer, sur la base d'une mesure d'impédance bioélectrique, les valeurs de certains paramètres de la composition corporelle, tels que la masse grasse, la masse maigre, la masse musculaire ou le contenu d'eau dans les tissus. L'appareil peut comporter par ailleurs un dispositif de mesure du poids, de la taille, de la taille de l'abdomen ou des hanches, du pouls, voire, il peut comporter un dispositif d'entrée ou de réception de ces données corporelles personnelles qui sont alors mesurées avec des appareils spécifiques à distance ou indépendants.

L'appareil de l'invention est alors apte à comparer les valeurs courantes des deux paramètres qui caractérisent la composition corporelle d'un individu à des valeurs antérieures préenregistrées de ces mêmes paramètres. Les valeurs des deux paramètres sont établies par l'appareil de manière concomitante ou simultanée pour les deux paramètres, c'est-à-dire lors d'une même prise de mesure. L'appareil procède ensuite à l'analyse conjointe de ces résultats afin d'en déduire si les deux paramètres évoluent de la même manière ou si l'un des paramètres devance l'autre et en quelle proportion. Il a été ainsi établi qu'en analysant la corrélation des évolutions des deux paramètres différents on arrive à caractériser de manière plus précise la tendance d'évolution de la composition corporelle d'un individu.

Selon l'invention, cette tendance d'évolution est communiquée à l'utilisateur par un message unique caractérisant l'évolution conjointe desdits paramètres. Ainsi, l'utilisateur est informé de manière explicite et non ambiguë sur l'efficacité d'un régime qu'il suit ou sur l'adéquation de son comportement alimentaire et/ou sportif entre deux mesures effectuées avec l'appareil de l'invention.

Avantageusement, l'un des paramètres de la composition corporelle est choisi dans le groupe comprenant le poids et la masse grasse et l'autre paramètre est choisi dans le groupe comprenant : la masse grasse, la masse maigre, la masse musculaire ou la masse hydrique.

On aurait pu, certes, choisir une autre combinaison de deux paramètres caractérisant la composition corporelle, notamment toute combinaison de deux paramètres choisis dans le groupe formé par : le poids, la masse grasse, la masse maigre, la masse musculaire, le contenu d'eau dans l'organisme, la masse osseuse, la masse grasse des viscères, le contenu de la masse grasse sous-cutanée.

Toutefois, lors de tests et analyses effectués, il a été constaté que les paramètres qui caractérisent mieux la composition corporelle sont, d'une part le poids comparé à la masse grasse ou à la masse maigre ou à la masse musculaire ou à la masse hydrique et, d'autre part, la masse grasse comparée à la masse maigre, à la masse musculaire ou à la masse hydrique d'un individu. Ainsi, en partant de deux de ces paramètres, il a été observé, notamment dans le cadre d'un régime non équilibré, qu'avec la diminution du poids, la masse musculaire diminuait en proportion plus importante que la masse grasse. On en déduit qu'il est important de connaître comment la relation qui lie ces deux paramètres évolue dans le temps afin d'en déduire une conduite à suivre soit dans le cadre d'un régime, soit pour maintenir sa forme. Plus particulièrement s'agissant de deux paramètres à suivre, cette relation se résume à déterminer leur corrélation dans les cas suivants l'un des paramètres est en augmentation ou en diminution, l'autre paramètre est en augmentation ou en diminution ou au moins l'un des deux est stable.

Pour des raisons de simplicité constructive et d'explication du fonctionnement de l'appareil, les paramètres choisis sont le poids et la masse grasse, dont l'interaction est très représentative de l'évolution de la composition corporelle de l'individu. Cette corrélation est alors réalisée par les moyens d'analyse de l'appareil qui transmettent le résultat aux moyens de communication qui informent l'utilisateur, par un seul message, que notamment, par rapport aux mesures précédentes, il a gagné en masse musculaire et en poids, ou il a pris du poids et de la masse grasse, ou il suit un régime non adapté ou il est en train de perdre de la masse grasse et du poids ou encore que sa composition corporelle est restée stable. En partant de cette analyse, des messages quantifiant ou précisant davantage ces tendances peuvent également être transmis par les moyens de communication.

Lesdits moyens de communication de l'appareil selon l'invention comprennent un dispositif d'affichage lumineux comportant une zone centrale et au moins une zone périphérique d'affichage dudit message.

On aurait pu, certes, utiliser un dispositif d'affichage numérique des valeurs calculées issues de la comparaison ou un avertisseur sonore apte à annoncer la tendance. On préfère toutefois un afficheur lumineux, car il est plus facilement compréhensible par l'utilisateur, tout en restant discret en fonctionnement. Par ailleurs, l'attention de l'utilisateur qui regarde un tel afficheur visuel est dirigée en premier vers la zone centrale qui peut alors être une zone cible, les messages dans la zone périphérique étant regardés par la suite. Bien que le dispositif d'affichage puisse être du type LCD, on préfère un afficheur lumineux car il s'est avéré qu'un effet lumineux auquel on attribue un code de couleurs arrive à être interprété plus facilement par l'utilisateur. Un tel dispositif d'affichage lumineux peut alors utiliser des LED ou des lampes électroluminescentes, etc.

La zone centrale correspond à une zone de stabilité conjointe des résultats.

En définissant une zone de stabilité au centre de l'afficheur, elle devient zone de référence pour l'utilisateur qui peut alors suivre l'évolution des paramètres de sa composition corporelle dont les valeurs seront placées par rapport à cette zone d'origine.

La zone périphérique correspond à une zone de variation des résultats.

Ceci permet alors de donner une appréciation visuelle très suggestive de l'écart par rapport à une zone centrale stable.

De préférence, ladite zone centrale est circulaire et elle est entourée d'au moins une zone annulaire d'affichage dudit message.

On aurait pu imaginer une autre représentation graphique comportant une zone centrale carrée ou rectangulaire avec un pourtour de même forme ou différente, par exemple circulaire. Un afficheur à zones concentriques s'est toutefois avéré plus compréhensible et plus facile à interpréter, tout en étant plus compact et facile à intégrer, par exemple, dans un afficheur de type LCD.

Utilement, ladite zone annulaire comprend quatre cadrans.

On aurait pu certes utiliser un anneau concentrique à la zone centrale de stabilité qui aurait pu représenter, par exemple à l'aide de deux couleurs, la tendance d'évolution des deux paramètres conjointement. Dans une variante, on aurait pu utiliser une représentation à deux cadrans, un cadran correspondant à chaque paramètre mesuré. On préfère toutefois une représentation à quatre cadrans, car elle est plus fine et, en même temps, représentative d'un affichage graphique basé sur une représentation en plan de la variation simultanée de deux paramètres en utilisant un système orthogonal d'axes de représentation, un axe étant alors attribué à chaque paramètre. L'intersection des axes de coordonnées (de représentation des paramètres poids et masse grasse) se trouverait alors au centre de la zone stable, les signes des différences obtenues par les moyens de comparaison pouvant être facilement placés dans l'un des quatre cadrans. Ceci simplifie le fonctionnement des moyens de calcul et d'analyse de l'appareil, tout en facilitant l'interprétation des résultats par l'utilisateur, les signes des différences pouvant alors également être affichés à l'intérieur de chaque cadran, à côté du nom du paramètre suivi.

Dans une réalisation avantageuse de l'appareil de l'invention, celui-ci comprend un plateau de mesure supporté par au moins un capteur de poids, la face supérieure du plateau comportant des électrodes destinées au contact des pieds et ledit dispositif d'affichage est situé sensiblement au centre dudit plateau.

Cette solution permet d'utiliser au mieux la surface du plateau de mesure de l'appareil et d'en réduire l'encombrement, le dispositif d'affichage étant alors disposé entre les électrodes, sur l'axe médian et de préférence au centre du plateau. Le dispositif d'affichage pouvant alors être du type LCD ou du type à LED, etc.

Avantageusement, lesdits moyens de comparaison réalisent des différences entre la dernière valeur mesurée pour chacun des deux paramètres différents de la composition corporelle et au moins une valeur antérieure mémorisée pour chaque paramètre.

Les moyens de comparaison auraient pu également fonctionner sur la base du calcul du rapport ou du pourcentage de variation de la valeur courante par rapport à la valeur antérieure. On préfère toutefois calculer des différences, car leurs valeurs sont plus explicites pour l'interprétation des résultats par les moyens d'analyse et s'appliquent plus facilement à une représentation utilisant un système orthogonal d'axes de coordonnées, où la zone de stabilité est placée dans l'origine, chaque axe représentant la variation d'un paramètre de la composition corporelle.

De préférence, lesdites valeurs antérieures sont la moyenne du poids et la moyenne de la masse grasse calculées sur la base de plusieurs mesures consécutives.

Dans un premier mode de réalisation de l'invention, par valeur antérieure mémorisée pour chaque paramètre on comprend une valeur brute mesurée par l'appareil notamment pour le poids, voire la valeur brute de la masse grasse calculée par l'appareil basée sur la valeur mesurée de l'impédance bioélectrique de l'individu.

Dans un mode préféré plus évolué de réalisation de l'invention, la valeur antérieure prise comme référence pour chaque paramètre est une valeur plus finement calculée par l'appareil en utilisant les moyennes de plusieurs mesures consécutives, par exemple sur une durée égale ou supérieure à sept jours, car elle est plus représentative de la valeur réelle du paramètre mesuré. La moyenne calculée peut être fixe, en étant par exemple calculée une fois pour toutes sur la base des premières mesures; ou elle peut être une moyenne glissante, la moyenne étant alors calculée sur une période de référence, mais qui est automatiquement décalée à chaque nouvelle prise de mesure.

Dans une première variante de réalisation de l'invention, lesdits moyens de comparaison commandent l'alimentation de la zone de stabilité de l'afficheur lorsque lesdites différences sont soit nulles, soit comprises dans des limites préétablies fixes de valeurs associées à chaque paramètre.

Cette variante permet, de manière simplifiée, de mettre en évidence la zone de stabilité dès lors que les différences effectuées par les moyens de calcul de l'appareil entre deux mesures consécutives de chaque paramètre ou alors entre deux moyennes consécutives de chaque paramètre sont nulles ou comprises dans des limites fixes stockées dans la mémoire de l'appareil au moment de sa fabrication. A titre d'exemple, ces limites peuvent être +/- 300g pour le poids et +/- 200g pour la masse grasse.

Dans une deuxième variante de réalisation de l'invention, lesdits moyens de comparaison commandent l'alimentation de la zone de stabilité de l'afficheur lorsque lesdites différences sont soit nulles, soit comprises dans des limites préétablies variables en fonction du poids et de la masse grasse de l'individu.

Ainsi, les moyens de calcul de l'appareil calculent les limites attribuées à chaque utilisateur en fonction de sa corpulence, donc en fonction du poids et de la masse grasse initialement calculés pour chaque utilisateur. Ces limites sont alors mémorisées pour chaque utilisateur et sont ensuite utilisées pour situer l'évolution de chaque paramètre. Cette solution présente l'avantage d'accepter des variations plus ou moins importantes des deux paramètres, en les adaptant au profil de l'utilisateur.

Dans une troisième variante de réalisation de l'invention, lesdits moyens de comparaison commandent l'alimentation de la zone de stabilité de l'afficheur lorsque lesdites différences sont soit nulles, soit comprises dans des limites préétablies variables adaptées aux fluctuations habituelles du poids et de la masse grasse de l'individu.

Les moyens de calcul de l'appareil modifient ainsi lesdites limites à chaque pesée en éliminant les fluctuations habituelles du poids et de la masse grasse de l'utilisateur. Ces fluctuations habituelles peuvent être calculées par des méthodes de calcul statistique d'un échantillon de plusieurs mesures.

Avantageusement, lesdits moyens de calcul déterminent également les écarts-types respectifs pour le poids et la masse grasse.

Il a été constaté que le suivi dans le temps du poids ou de la masse grasse fait apparaître des fluctuations importantes de ces paramètres. Il est alors important de soustraire les fluctuations aléatoires de ces paramètres afin de bien pouvoir interpréter par la suite les tendances d'évolution. Les fluctuations aléatoires des paramètres peuvent être délimitées en utilisant des mesures de dispersion utilisées en statistique, notamment l'écart-type qui est utile pour mesurer la dispersion autour de la moyenne d'un ensemble de données. Ainsi, on arrive à bien délimiter un intervalle de stabilité d'un paramètre en calculant des écart-types pour chaque paramètre et en fixant, de préférence, des limites de cet intervalle de stabilité à plus ou moins trois écart-types autour de la valeur moyenne pour chaque paramètre. Une valeur mesurée qui sort de ces limites pourrait alors être considérée comme représentative de la tendance d'évolution du paramètre étudié.

De manière avantageuse, ladite zone de stabilité est déterminée sur la base des moyennes et des écarts-types calculés pour les deux paramètres.

La zone centrale de stabilité est une zone comprenant les valeurs mesurées du poids et de la masse grasse comprises entre plus ou moins trois écart-types autour de leur moyenne respective. Si l'on représente alors graphiquement sur un système d'axes de coordonnées avec, sur l'axe des abscisses le poids et sur l'axe des ordonnées la masse grasse et si l'on fixe l'origine de ce système au centre de la zone de stabilité, on remarque alors que les points qui sortent de cette zone sont disposés sur le pourtour de la zone de stabilité et que le signe de la différence par rapport aux valeurs limites de la zone de stabilité détermine leur positionnement dans l'un des cadrans du système d'axes de coordonnées. Cette représentation est à la base de l'afficheur graphique du mode préféré de réalisation de l'invention.

Dans un autre mode de réalisation de l'invention, l'appareil comprend un plateau de mesure supporté par au moins un capteur de poids, la face supérieure du plateau comportant des électrodes destinées au contact des pieds et un dispositif d'affichage du type LCD situé en la partie avant dudit plateau.

Ce dispositif d'affichage est alors plus discret et il doit être placé dans une partie plus facilement visible par l'utilisateur et de plus grande étendue pour plus de confort.

De préférence, lesdits moyens de communication comprennent un dispositif d'affichage numérique des résultats.

Il s'avère parfois préférable d'informer l'utilisateur de la valeur exacte du paramètre mesuré ou de ses limites de variation, raison pour laquelle on choisit d'équiper l'appareil d'un dispositif d'affichage numérique. Ce dispositif d'affichage numérique peut être ajouté au dispositif d'affichage graphique, en étant agencé à côté ou à distance d'un dispositif d'affichage graphique, voire même en étant intégré à ce dernier.

L'invention sera mieux comprise à l'étude des modes de réalisation pris à titre nullement limitatif et illustrés dans les figures annexées dans lesquelles:

- la figure 1 est une vue de dessus d'un appareil de mesure de la composition corporelle selon un mode préféré de réalisation de l'invention,
- la figure 2 est un schéma bloc des principales parties constitutives de l'appareil;
- la figure 3 est une représentation graphique des deux paramètres mesurés par l'appareil de la figure 1 montrant leurs possibilités d'évolution;
- la figure 4 représente une vue de dessus d'un dispositif d'affichage graphique selon une première variante de l'invention;
- la figure 5 représente une vue de dessus d'un dispositif d'affichage graphique selon une deuxième variante de l'invention;
- la figure 6 représente une vue de dessus d'un dispositif d'affichage graphique selon un mode préféré de réalisation de l'invention;
- la figure 7 représente une vue de dessus d'un dispositif d'affichage selon un autre mode de réalisation de l'invention.

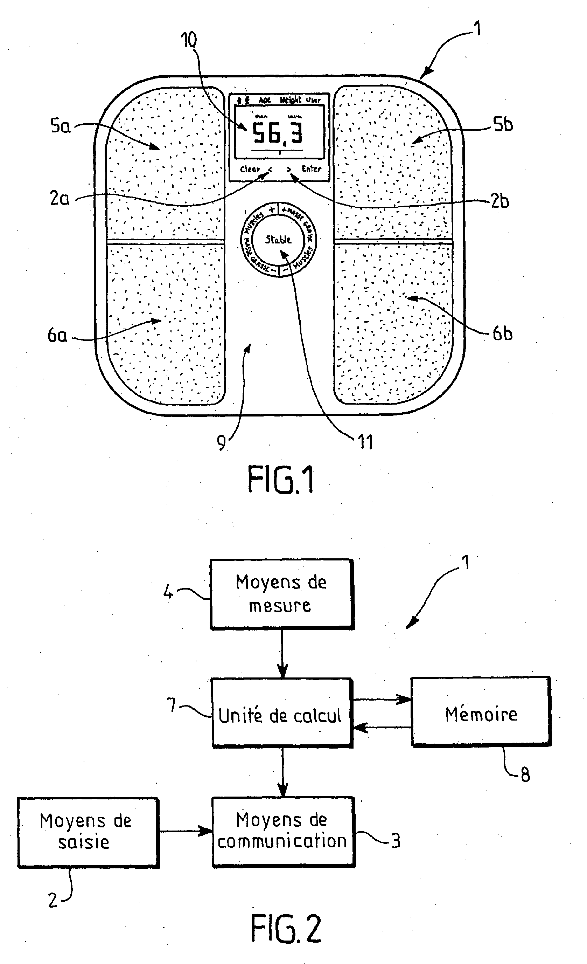

La figure 1 illustre un exemple d'appareil de mesure 1 de la composition corporelle d'un individu basé sur la mesure d'une impédance bioélectrique. L'appareil comprend un plateau 9 de mesure muni sur sa face supérieure de quatre électrodes et supporté par des pieds renfermant des capteurs de poids. Les capteurs de poids comprennent chacun un barreau muni de jauges de contrainte reliées en pont de Wheatstone, les quatre capteurs étant connectés ensemble dans un circuit commun qui envoie le signal de mesure, via un convertisseur A/D, à une unité de calcul 7 et à une mémoire 8. Le résultat de la pesée est ensuite envoyé aux moyens de communication 3 qui le font apparaître à l'écran d'un dispositif d'affichage numérique 10.

Le plateau 9 comporte deux électrodes d'excitation 5a,5b reliées à un générateur de courant ou de tension qui applique un signal d'excitation entre deux points du corps de l'individu, notamment à la partie avant de ses pieds. Le plateau 9 comporte également deux électrodes de mesure 6a,6b d'une différence de potentiel entre les talons de l'utilisateur après passage du signal d'excitation appliqué à travers son corps. L'appareil de mesure 1 comporte par ailleurs des moyens de saisie 2 des données corporelles personnelles sous forme d'un clavier muni de boutons de sélection 2a,2b des valeurs spécifiques à l'individu qui sont ensuite affichées à l'écran du dispositif d'affichage numérique 10.

A la figure 2 est représenté un schéma bloc de fonctionnement d'un tel appareil où les moyens de mesure 4 sont constitués par les capteurs de poids et les électrodes de l'appareil dont les circuits électriques transmettent les signaux mesurés à une unité de calcul 7, notamment un microprocesseur ou microcontrôleur adaptés au traitement à effectuer. L'unité de calcul 7 est reliée à une mémoire 8 apte à stocker les données spécifiques à l'individu, telles les données mesurées par les moyens de mesure 4, ainsi que les données corporelles personnelles introduites par l'utilisateur par des moyens de saisie 2. Les valeurs calculées par l'unité de calcul 7 ou stockées dans la mémoire 8 sont affichées par des moyens de communication 3. Ces valeurs peuvent être le poids, la quantité de masse grasse, la quantité de masse maigre, la masse musculaire, le BMI (rapport entre le poids et le carré de la taille), le contenu d'eau de l'organisme, ou tout autre paramètre calculé sur la base de l'impédance bioélectrique du corps de l'individu.

L'unité de calcul 7 de l'appareil de l'invention comprend des moyens de comparaison des valeurs courantes mesurées pour deux paramètres différents caractérisant la composition corporelle de l'individu, valeurs issues des signaux transmis par les moyens de mesure 4, avec des valeurs antérieures mesurées, calculées et stockées dans la mémoire 8 de l'appareil. Plus particulièrement selon l'invention, l'unité de calcul 7 comprend des moyens d'analyse des résultats issus des moyens de comparaison des deux paramètres simultanés qui envoient un signal aux moyens de communication 3, signal qui est alors représentatif de l'évolution globale de la composition corporelle de l'individu.

La comparaison d'une valeur courante avec une valeur antérieure mémorisée a pour but d'en déduire la tendance d'évolution du paramètre suivi. Or, il s'est avéré que les paramètres de la composition corporelle, tels le poids et la masse grasse ou la masse maigre, subissent des fluctuations importantes d'un jour à l'autre, sans qu'une tendance de prise ou de perte de poids ou de masse grasse se soit bien installée. Ces fluctuations sont dues à des facteurs tels que l'activité physique, le stress, le cycle hormonal, des excès alimentaires ponctuels, etc. Il est alors important de soustraire ces fluctuations des enregistrements effectués sur la base des mesures successives, par exemple journalières, et de comparer alors la valeur courante à une valeur antérieure qui soit représentative de la tendance d'évolution du paramètre mesuré.

Dans un mode préféré de réalisation de l'invention, la valeur antérieure prise en compte pour la comparaison avec la valeur courante, notamment pour le poids et la masse grasse, est la moyenne des N poids mesurés sur une durée supérieure ou égale à sept jours et la moyenne des N valeurs de la masse grasse enregistrées sur une même période. Afin d'extraire les fluctuations aléatoires des valeurs enregistrées, l'unité de calcul 7 effectue le calcul des écart-

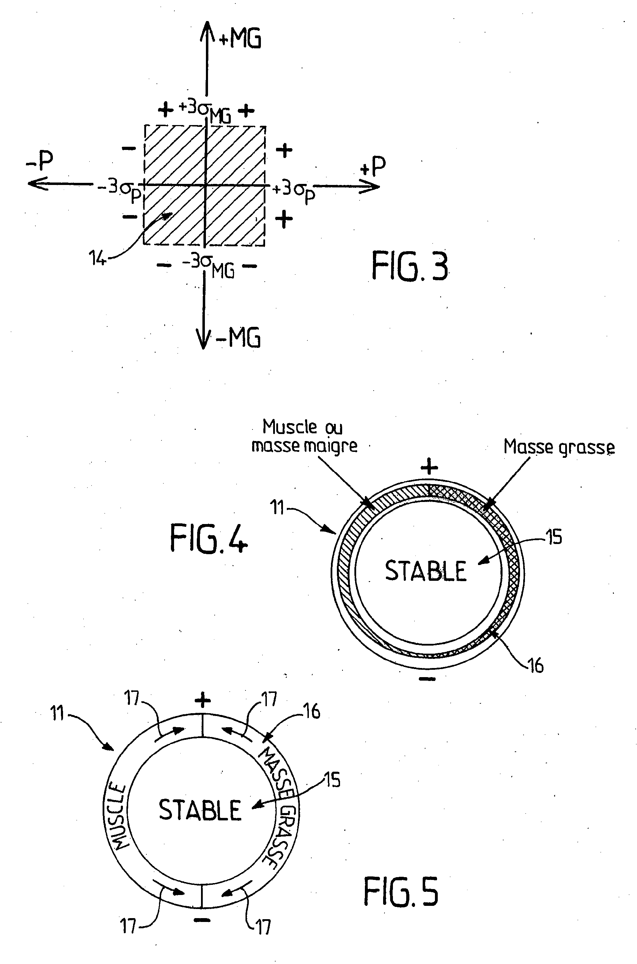

types $\sigma_P$ pour les N valeurs mesurées du poids et les écart-types $\sigma_{MG}$ pour les N valeurs mesurées de la masse grasse. Avec les résultats obtenus, il est possible de représenter alors graphiquement le poids et la masse grasse de l'individu pour une mesure N+1 sur des axes de coordonnées, tel que visible à la figure 3.

La figure 3 est une représentation graphique du poids et de la masse grasse de l'individu, ces deux données évoluant dans le temps, avec la masse grasse MG sur l'axe des ordonnées et le poids P sur l'axe des abscisses. Dans une variante, on peut envisager une représentation graphique de ces deux paramètres en mettant le poids sur l'axe des ordonnées et la masse grasse sur celui des abscisses.

De manière avantageuse selon un mode preféré de l'invention, on calcule la moyenne du poids et celle de la masse grasse et on délimite, sur chaque axe, les plus et moins trois écart-types. La zone hachurée de la figure 3 forme ainsi une zone de stabilité 14 en étant délimitée, sur l'axe des ordonnées, par les $-3\sigma_{MG}$ et $+3\sigma_{MG}$ et, sur l'axe des abscisses, par par les $-3\sigma_P$ et $+3\sigma_P$, l'origine du système des axes correspondant alors à la valeur moyenne calculée pour la masse grasse et pour le poids pour les N valeurs précédentes. La valeur mesurée suivante (N+1) pour la masse grasse peut alors être représentée sur l'axe des ordonnées et on effectue ensuite la différence avec la valeur moyenne de la masse grasse calculée pour les N mesures précédentes $+/-3\sigma_{MG}$, le signe de cette différence étant représenté sur le graphique de la figure 3. On procède de la même manière avec la mesure suivante N+1 pour le poids et on calcule la différence par rapport à la valeur moyenne du poids calculée pour les N mesures précédentes $+/-3\sigma_P$. Ainsi, ces opérations effectuées, on détermine le sens de variation de chaque paramètre par rapport aux mesures précédentes. Une fois le sens de variation défini, les moyens d'analyse envoient un signal aux moyens de communication pour signifier d'une manière aisément compréhensible la tendance d'évolution des deux paramètres simultanément.

Ainsi, au cas où les deux valeurs P et MG de la mesure N+1 tombent à l'intérieur de la zone de stabilité, le message "stable" est transmis par les moyens de communication 3. Dans une variante non revendiquée, on peut choisir de ne rien communiquer à l'utilisateur qui comprendra que, au cas où il n'y a pas de message, les mesures courantes ne varient pas par rapport à la zone de stabilité délimitée.

Pour tout point hors la zone de stabilité 14, en étudiant les signes des différences calculées, on peut définir dans quel cadran de la représentation graphique de la figure 3 appartient le nouveau point et ainsi définir la tendance de l'évolution des deux paramètres conjoints : poids et masse grasse. Les moyens d'analyse transmettent alors ce message aux moyens de communication qui l'annoncent à l'utilisateur.

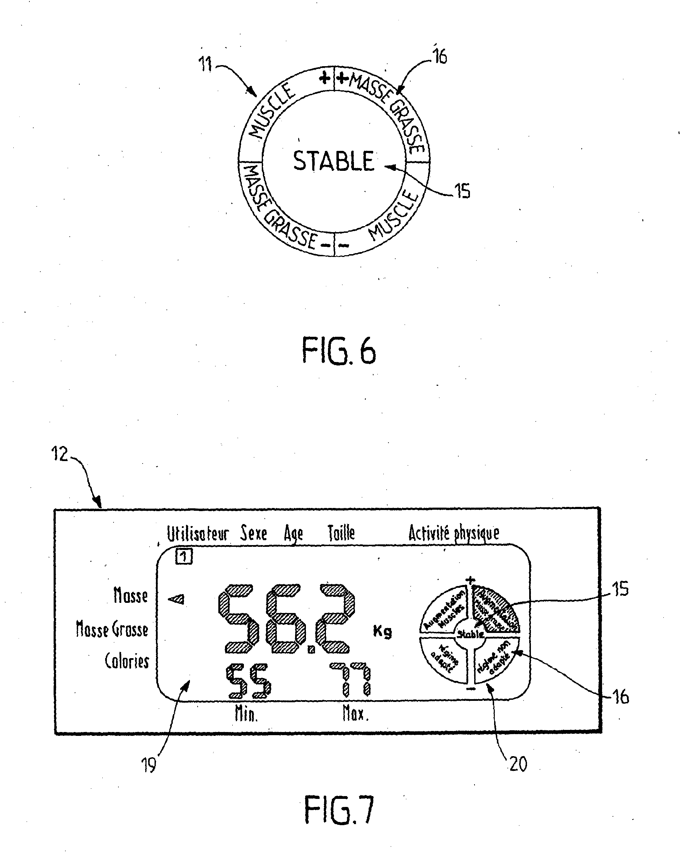

Selon un mode préféré de réalisation de l'invention, les moyens de communication du résultat de l'analyse sont constitués par un dispositif d'affichage graphique 11 (fig. 1 et 6) disposé au centre du plateau 9. Ce dispositif d'affichage comprend une zone centrale 15 circulaire, correspondant à la zone de stabilité 14, entourée d'une zone périphérique 16 annulaire découpée en quatre cadrans, chacun des cadrans correspondant à un type d'évolution conjointe des paramètres analysés. L'ensemble de la zone du dispositif d'affichage 11 reste visible même lorsque le dispositif est éteint, des mots ou messages pouvant être gravés ou imprimés sur une partie transparente ou fenêtre du dispositif d'affichage 11, éventuellement sur un fond coloré de cette dernière. Ainsi, sur un premier cadran situé en haut à gauche par rapport à la zone centrale 15 peut être inscrit le mot "muscle" suivi d'un signe "+", sur le cadran suivant, situé en haut à droite, est inscrit le mot "masse grasse" suivi du signe "+", sur le cadran suivant situé en bas à droite, peut être inscrit le mot "muscle" suivi de signe "-" et enfin le dernier cadran situé en bas à gauche présente le mot "masse grasse" suivi du signe "-". En fonctionnement, à chaque pesée, une fois l'analyse effectuée, un segment de forme correspondante à celle du cadran concerné vient s'afficher en superposition totale ou partielle avec la zone visible du dispositif d'affichage dans le cas où ledit dispositif d'affichage est réalisé à l'aide d'un afficheur LCD. Dans le cas où l'on utilise un dispositif lumineux à LED, la ou les LED correspondantes au cadran concerné s'allument.

Le dispositif d'affichage représenté à la figure 6 comporte, insérées dans la zone du plateau située en dessous, des lampes qui viennent éclairer la zone adéquate de manière à la mettre en évidence par rapport aux autres zones non concernées. Ces lampes peuvent être des LED ayant différentes couleurs, par exemple, rouge pour les deux cadrans situés à droite, verte pour les deux cadrans situés à gauche. La zone centrale 15 peut être affichée par une couleur neutre, jaune par exemple, ou elle peut ne pas comporter de lampe d'indication lumineuse.

La figure 5 représente une variante du dispositif d'affichage graphique de la figure 1, mais où la zone périphérique 16 est divisée en deux cadrans, le cadran de gauche pouvant alors représenter l'évolution de la masse maigre ou de la masse musculaire et le cadran de droite celui de la masse grasse. Un sens de l'évolution est donné par des flèches 17 qui peuvent être mises en évidence par des lampes qui s'allument en dessous. Ainsi, en fonction du résultat de la mesure, soit la zone centrale de stabilité 15 s'illumine, soit une autre lampe parmi la série de lampes équidistantes formant la zone périphérique 16.

La figure 4 représente un dispositif d'affichage comportant une zone centrale 15 de stabilité entourée d'une zone périphérique 16 comportant des zones hachurées différemment et ayant inscrits les sens d'évolution (+,-) de la masse musculaire ou masse maigre à gauche et de la masse grasse à droite. De la même manière qu'à la figure 5, en fonction du résultat de la mesure, soit la zone centrale de stabilité 15 s'illumine, soit une autre lampe parmi la série de lampes équidistantes formant la zone périphérique 16.

Les dispositifs d'affichage 11 illustrés dans les figures 4 à 6 sont des dispositifs lumineux du type comportant des LED ou des lampes électroluminescentes contenant une couche de phosphore prise en sandwich entre une première couche conductrice transparente supérieure et, moyennant un diélectrique, une deuxième couche conductrice inférieure. Ces lampes électroluminescentes peuvent être déposées, par exemple par sérigraphie des différentes couches les composant, sur un film rapporté sur le plateau ou directement sur le plateau de l'appareil. Dans une variante de l'invention, ces dispositifs peuvent être des afficheurs LCD.

Les dispositifs d'affichage graphique 11 selon les figures 4 à 6 peuvent être combinés avec des dispositifs d'affichage numériques 10, tel que représenté à la figure 1. Ces dispositifs d'affichage numériques 10 affichent initialement l'information relative à l'utilisateur (ses données corporelles personnelles), et par la suite, le résultat de chaque pesée et éventuellement ses limites minimum et maximum de variation.

La figure 7 illustre un autre dispositif d'affichage 12 selon un autre mode de réalisation de l'invention. Ce dispositif d'affichage comporte une zone numérique 19 et une zone graphique 20 d'affichage. Les deux zones numérique 19 et graphique 20 peuvent être intégrées dans un afficheur du type LCD. La zone numérique 19 affiche le résultat de la pesée, les unités de mesure, ainsi que les limites de variation minimum et maximum ou de normalité pour un individu. La zone graphique 20 affiche, de la même manière que précédemment, une zone circulaire de stabilité 15 entourée d'une zone périphérique 16 affichant la tendance d'évolution des paramètres conjoints. Un message peut également être inscrit (gravé ou imprimé) sur chacun des cadrans de la zone annulaire 16 de la fenêtre de l'afficheur et un segment de l'afficheur LCD vient s'afficher en superposition totale ou partielle de la zone du cadran concerné. A titre d'exemple, ces messages peuvent être : une augmentation de la masse musculaire de l'utilisateur dans le cadran de la zone périphérique 16 qui est situé en haut à gauche par rapport à la zone centrale 15 de stabilité; une augmentation de sa masse grasse dans le cadran situé en haut à droite; ou le fait que l'utilisateur suit un régime non adapté qui est alors illustré par le cadran situé en bas à droite; ou encore que son régime est bien adapté ou équilibré, illustré par le cadran situé en bas à gauche de la zone centrale 15.

Bien qu'une zone périphérique 16 à un, deux ou quatre cadrans ait été illustrée aux figures, on pourrait également envisager une zone périphérique à plusieurs cadrans qui serait alors plus finement divisée, chacun des cadrans étant prévu pour communiquer des indications encore plus précises à l'utilisateur sur la corrélation entre les deux paramètres observés. Ainsi, plusieurs cadrans peuvent composer une zone d'affichage graphique d'un afficheur LCD ou d'un dispositif lumineux, et dans ce cas l'affichage pourrait se faire par plusieurs lampes colorées, voire par des lampes qui, en combinaison avec la fenêtre de la zone périphérique 16 présenteraient un dégradé de couleurs.

Dans une variante simplifiée de réalisation de l'invention, les moyens de calcul de l'appareil calculent les limites attribuées à chaque utilisateur en fonction de sa corpulence, donc en fonction du poids et de la masse grasse initialement calculés pour chaque utilisateur. Ces limites sont alors mémorisées pour chaque utilisateur et sont ensuite utilisées pour situer l'évolution de chaque paramètre. Les limites ou seuils pour le poids et pour la masse grasse sont alors calculés avec les formules :

Seuil 1 = K1 x poids
Seuil 2 = K2 x masse grasse
Où K1 <1 et K2<1.

Cette solution présente l'avantage d'accepter des variations plus ou moins importantes des deux paramètres, en les adaptant au profil de l'utilisateur.

Dans une autre variante encore plus simple, les limites ou seuils utilisés pour la comparaison sont des valeurs fixes stockées dans la mémoire de l'appareil au moment de sa fabrication. La valeur de la différence peut alors être nulle ou comprise entre des limites préétablies. A titre d'exemple, ces limites peuvent être +/- 0.3kg pour le poids et +/- 0.2kg pour la masse grasse.

Les calculs s'effectuent alors avec la formule :

$$(\text{poids (n) - poids (n-1)} < \text{seuil 1}) \text{ ET (masse grasse (n) - masse grasse (n-1))} < \text{seuil 2})$$

lorsque l'on utilise les valeurs mémorisées lors de la prise de mesure précédente; ou encore avec la formule :

$$(\text{poids (n) - moyenne de poids (n-1)} < \text{seuil 1}) \text{ ET (masse grasse (n) - moyenne de masse grasse (n-1))} < \text{seuil 2})$$

lorsque l'on utilise comme base de comparaison une moyenne des mesures précédentes.

D'autres variantes et modes de réalisation de l'invention peuvent être envisagés sans sortir du cadre de ces revendications.

Ainsi, pour définir la zone de stabilité, on pourrait utiliser d'autres méthodes de calcul ou d'analyse statistique des données mesurées.

## Revendications

1. Appareil de mesure de la composition corporelle comportant des moyens de mesure (4) d'une impédance bioélectrique, des moyens de mesure du poids, des moyens de saisie (2) des données corporelles personnelles, des moyens de mise en mémoire (8) et de calcul (7) des valeurs de la composition corporelle reliés à des moyens de communication (3) des résultats, où lesdits moyens de calcul comportent des moyens de comparaison qui comparent les dernières valeurs mesurées pour deux paramètres différents de la composition corporelle d'un individu à des valeurs antérieures de ces paramètres mesurées pour un même individu, **caractérisé en ce que** : lesdits moyens de communication comprennent un dispositif d'affichage (11, 12) lumineux comportant une zone centrale (15) et au moins une zone périphérique (16) d'affichage d'un message, les moyens de calcul comportent des moyens d'analyse des deux résultats issus des moyens de comparaison lesdits moyens d'analyse étant configurés pour envoyer un signal auxdits moyens de communication (3) pour émettre un message unique représentatif de l'évolution conjointe des deux paramètres, soit par illumination de la zone centrale (15) correspondant à une zone de stabilité conjointe (14) des résultats soit par illumination d'une zone périphérique (16) correspondant à une zone de variation des résultats.

2. Appareil selon la revendication 1, caractérisé en ce l'un des paramètres de la composition corporelle est choisi dans le groupe comprenant le poids et la masse grasse et l'autre paramètre est choisi dans le groupe comprenant : la masse grasse, la masse maigre, la masse musculaire ou la masse hydrique.

3. Appareil selon la revendication 1, **caractérisé en ce que** ladite zone centrale (15) est circulaire et qu'elle est entourée d'au moins une zone annulaire d'affichage dudit message.

4. Appareil selon la revendication précédente, **caractérisé en ce que** ladite zone annulaire comprend quatre cadrans.

5. Appareil selon l'une des revendications précédentes, **caractérisé en ce qu'**il comprend un plateau (9) de mesure supporté par au moins un capteur de poids, la face supérieure du plateau (9) comportant des électrodes (5a,5b,6a, 6b) destinées au contact des pieds et **en ce que** ledit dispositif d'affichage (11) est situé sensiblement au centre dudit plateau (9).

6. Appareil selon l'une des revendications précédentes, **caractérisé en ce que** lesdits moyens de comparaison réalisent des différences entre la dernière valeur mesurée pour chacun des deux paramètres différents de la composition corporelle et au moins une valeur antérieure mémorisée pour chaque paramètre.

7. Appareil selon la revendication précédente, **caractérisé en ce que** lesdites valeurs antérieures sont la moyenne du poids et la moyenne de la masse grasse calculées sur la base de plusieurs mesures consécutives.

8. Appareil selon l'une des revendications 6 à 7, **caractérisé en ce que** lesdits moyens de comparaison commandent l'alimentation de la zone de stabilité (14) de l'afficheur lorsque lesdites différences sont soit nulles, soit comprises dans des limites préétablies fixes de valeurs associées à chaque paramètre.

9. Appareil selon l'une des revendications 6 à 7, **caractérisé en ce que** lesdits moyens de comparaison commandent l'alimentation de la zone de stabilité (14) de l'afficheur lorsque lesdites différences sont soit nulles, soit comprises dans des limites préétablies variables en fonction du poids et de la masse grasse de l'individu.

10. Appareil selon l'une des revendications 6 à 7, **caractérisé en ce que** lesdits moyens de comparaison commandent l'alimentation de la zone de stabilité (14) de l'afficheur lorsque lesdites différences sont soit nulles, soit comprises dans des limites préétablies variables adaptées aux fluctuations habituelles du poids et de la masse grasse de l'individu.

11. Appareil selon la revendication précédente, **caractérisé en ce que** lesdits moyens de calcul sont prévus pour calculer les écarts-types respectifs pour le poids et la masse grasse.

12. Appareil selon la revendication précédente, **caractérisé en ce que** ladite zone de stabilité (14) est déterminée sur la base des moyennes et des écarts-types calculés pour les deux paramètres.

13. Appareil selon l'une des revendications précédentes, **caractérisé en ce qu'**il comprend un plateau (9) de mesure supporté par au moins un capteur de poids, la face supérieure du plateau (9) comportant des électrodes (5a,5b,6a, 6b) destinées au contact des pieds et **en ce qu'**il comporte un dispositif d'affichage (12) du type LCD situé en la partie avant dudit plateau.

14. Appareil selon l'une des revendications précédentes, **caractérisé en ce que** lesdits moyens de communication (3) comprennent un dispositif d'affichage numérique (10) des résultats.

## Patentansprüche

1. Gerät zur Messung der Körperzusammensetzung mit Vorrichtungen zur Messung (4) der bioelektrischen Impedanz, Vorrichtungen zur Gewichtsmessung, Vorrichtungen zur Eingabe (2) persönlicher Körperdaten sowie Vorrichtungen zur Speicherung (8) und Berechnung (7) von Werten der Körperzusammensetzung, die mit Vorrichtungen zur Ausgabe (3) der Ergebnisse verbunden sind, wobei die genannten Berechnungsvorrichtungen Vergleichsmittel umfassen, die die zuletzt für zwei verschiedene Parameter der Körperzusammensetzung einer Person gemessenen Werte mit zu einem früheren Zeitpunkt für dieselbe Person gemessenen Werten dieser Parameter vergleichen, **dadurch gekennzeichnet, dass** die genannten Ausgabevorrichtungen eine Leuchtanzeige (11, 12) mit einem zentralen Textanzeigebereich (15) und mindestens einem peripheren Textanzeigebereich (16) umfassen und dass die Berechnungsvorrichtungen mit Mitteln zur Analyse von zwei Ergebnissen der Vergleichsmittel ausgestattet sind, wobei diese Analysemittel so konfiguriert sind, dass sie ein Signal an die genannten Ausgabevorrichtungen (3) senden, damit eine einzige Anzeige ausgegeben wird, die die gemeinsame Entwicklung der beiden Parameter darstellt, und zwar entweder durch die Beleuchtung des zentralen Bereichs, der einem Bereich entspricht, in dem eine gemeinsame Stabilität der Ergebnisse vorherrscht (14), oder durch Beleuchtung eines peripheren Bereichs (16), der einem Bereich mit abweichenden Ergebnissen entspricht.

2. Gerät nach Anspruch 1, **dadurch gekennzeichnet, dass** einer der Parameter der Körperzusammensetzung aus der das Gewicht und die Fettmasse umfassenden Gruppe und der andere Parameter aus der die Fettmasse, die Magermasse, die Muskelmasse und das Körperwasser umfassenden Gruppe ausgewählt wird.

3. Gerät nach Anspruch 1, **dadurch gekennzeichnet, dass** der zentrale Bereich (15) kreisförmig gestaltet und von mindestens einem ringförmigen Bereich für die genannte Anzeige umgeben ist.

4. Gerät nach dem vorhergehenden Anspruch, **dadurch gekennzeichnet, dass** der genannte ringförmige Bereich vier Skalenbereiche umfasst.

5. Gerät nach einem der vorhergehenden Ansprüche, **dadurch gekennzeichnet, dass** es eine Messplatte (9) umfasst, die auf mindestens einem Gewichtssensor aufliegt, wobei die Oberseite der Platte (9) mit Elektroden (5a, 5b, 6a, 6b) versehen ist, die für den Kontakt mit den Füßen bestimmt sind, und **dadurch gekennzeichnet, dass** die genannte Anzeige (11) im Wesentlichen in der Mitte dieser Platte (9) angeordnet ist.

6. Gerät nach einem der vorhergehenden Ansprüche, **dadurch gekennzeichnet, dass** die genannten Vergleichsmittel die Differenzen zwischen dem zuletzt gemessenen Wert der beiden unterschiedlichen Parameter der Körperzusammensetzung und mindestens einem für jeden dieser Parameter gespeicherten früheren Wert ermitteln.

7. Gerät nach dem vorhergehenden Anspruch, **dadurch gekennzeichnet, dass** die genannten früheren Werte dem durchschnittlichen Gewicht und der durchschnittlichen Fettmasse entsprechen, die auf der Grundlage verschiedener aufeinanderfolgender Messungen berechnet wurden.

8. Gerät nach einem der Ansprüche 6 oder 7, **dadurch gekennzeichnet, dass** die Vergleichsmittel die Stromversorgung des Stabilitätsbereichs (14) der Anzeige steuern, wobei die genannten Differenzen entweder Null betragen oder in die vorgegebenen festen Grenzbereiche der Werte fallen, die den einzelnen Parametern zugeordnet sind.

**9.** Gerät nach einem der Ansprüche 6 oder 7, **dadurch gekennzeichnet, dass** die Vergleichsmittel die Stromversorgung des Stabilitätsbereichs (14) der Anzeige steuern, wobei die genannten Differenzen entweder Null betragen oder in die vorgegebenen variablen Grenzbereiche fallen, die von Gewicht und Fettmasse der Person abhängig sind.

**10.** Gerät nach einem der Ansprüche 6 oder 7, **dadurch gekennzeichnet, dass** die Vergleichsmittel die Stromversorgung des Stabilitätsbereichs (14) der Anzeige steuern, wobei die genannten Differenzen entweder Null betragen oder in die vorgegebenen variablen Grenzbereiche fallen, die an die üblichen Schwankungen von Gewicht und Fettmasse der Person angepasst sind.

**11.** Gerät nach dem vorhergehenden Anspruch, **dadurch gekennzeichnet, dass** die Berechnungsvorrichtungen dazu dienen, die Standardabweichungen für Gewicht und Fettmasse zu berechnen.

**12.** Gerät nach dem vorhergehenden Anspruch, **dadurch gekennzeichnet, dass** der genannte Stabilitätsbereich (14) ausgehend von den für die beiden Parameter berechneten Durchschnittswerten und Standardabweichungen festgelegt wird.

**13.** Gerät nach einem der vorhergehenden Ansprüche, **dadurch gekennzeichnet, dass** es eine Messplatte (9) umfasst, die auf mindestens einem Gewichtssensor aufliegt, wobei die Oberseite der Platte (9) mit Elektroden (5a, 5b, 6a, 6b) versehen ist, die für den Kontakt mit den Füßen bestimmt sind, und **dadurch gekennzeichnet, dass** es mit einer im vorderen Bereich der Platte angeordneten LCD-Anzeige (12) ausgestattet ist.

**14.** Gerät nach einem der vorhergehenden Ansprüche, **dadurch gekennzeichnet, dass** die genannten Ausgabevorrichtungen (3) eine Digitalanzeige (10) für die Ausgabe der Ergebnisse umfassen.

## Claims

**1.** Appliance for measuring the body composition comprising means for measuring (4) a bioelectrical impedance, means for measuring the weight, means for inputting (2) the personal body data, means for storing (8) and calculating (7) the values of the body composition connected to means for communicating (3) the results, wherein said calculating means comprise comparison means which compare the last values measured for two different parameters of an individual's body composition with previous values of these parameters measured for the same individual, **characterised in that**: said communication means include an illuminated display device (11, 12) comprising a central area (15) and at least one peripheral area (16) for displaying a message, the calculating means comprise means for analysing the two results from the comparison means, said analysis means being configured to send a signal to said communication means (3) for generating a single message representative of the joint evolution of the two parameters, either by illumination of the central area (15) corresponding to an area (14) of joint stability of the results or by illumination of a peripheral area (16) corresponding to an area of variation of the results.

**2.** Appliance according to claim 1, **characterised in that** one of the body composition parameters is selected from the group comprising the weight and body fat and the other parameter is selected from the group comprising: body fat, lean body mass, muscle mass or body water.

**3.** Appliance according to claim 1, **characterised in that** said central area (15) is circular and that it is surrounded by at least one annular area for displaying said message.

**4.** Appliance according to the preceding claim, **characterised in that** said annular area comprises four quadrants.

**5.** Appliance according to one of the preceding claims, **characterised in that** it comprises a measuring platform (9) supported by at least one weight sensor, the upper surface of the platform (9) comprising electrodes (5a, 5b, 6a, 6b) for contact with the feet, and **in that** said display device (11) is located substantially at the centre of said platform (9).

**6.** Appliance according to one of the preceding claims, **characterised in that** said comparison means measure the differences between the last value measured for each of the two different parameters of the body composition and at least one previous value stored for each parameter.

**7.** Appliance according to the preceding claim, **characterised in that** said previous values are the average weight and the average body fat calculated on the basis of several consecutive measurements.

8. Appliance according to claim 6 or 7, **characterised in that** said comparison means control the power supply of the display stability area (14) when said differences are either zero or lie between fixed predetermined limits of values associated with each parameter.

9. Appliance according to claim 6 or 7, **characterised in that** said comparison means control the power supply of the display stability area (14) when said differences are either zero or lie between variable predetermined limits depending on the individual's weight and body fat.

10. Appliance according to claim 6 or 7, **characterised in that** said comparison means control the power supply of the display stability area (14) when said differences are either zero or lie between variable predetermined limits adapted to the individual's usual weight and body fat fluctuations.

11. Appliance according to the preceding claim, **characterised in that** said calculating means are provided for calculating the respective standard deviations for weight and body fat.

12. Appliance according to the preceding claim, **characterised in that** said stability area (14) is determined on the basis of the averages and standard deviations calculated for the two parameters.

13. Appliance according to one of the preceding claims, **characterised in that** it comprises a measuring platform (9) supported by at least one weight sensor, the upper surface of the platform (9) comprising electrodes (5a, 5b, 6a, 6b) for contact with the feet, and **in that** it comprises an LCD type display device (12) located at the front of said platform.

14. Appliance according to one of the preceding claims, **characterised in that** said communication means (3) comprise a digital device (10) for displaying the results.

**FIG.1**

**FIG.2**

EP 1 827 223 B1

FIG.3

FIG.4

Muscle ou
masse maigre

Masse grasse

STABLE

FIG.5

MUSCLE

MASSE GRASSE

STABLE

14

FIG. 6

FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9952425 A
- EP 1201187 A
- US 20040199057 A
- EP 1386581 A